# EUROPEAN PATENT APPLICATION

(11) **EP 4 847 049 A1**
(43) Date of publication of application: **02.12.2026**
(21) Application number: 25767966.2
(22) Date of filing: 17.02.2025
(51) Int. Cl.: A61K 51/00, C07D 401/12, G01T 1/161

(54) **DIAGNOSTIC AGENT FOR CARDIAC FUNCTION**

(30) Priority: 06.03.2024 JP 2024033666
(71) Applicant: Hamamatsu Photonics K.K., Hamamatsu-shi, Shizuoka 435-8558 (JP)
(72) Inventor: OHBA, Hiroyuki, Hamamatsu-shi, Shizuoka 435-8558 (JP); TSUKADA, Hideo, Hamamatsu-shi, Shizuoka 435-8558 (JP)
(74) Representative: Grünecker Patent- und Rechtsanwälte PartG mbB
(86) International application number: PCT/JP2025/005200
(87) International publication number: WO 2025/187382

(57) **Abstract**

A diagnostic agent for cardiac function, containing a compound represented by General Formula (1-0) as an active component. 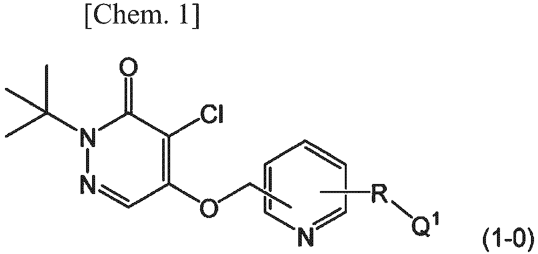 [In General Formula (1-0), R represents -O(CH₂)ₙ-, - O(CH₂)ₙOC₂H₄-, -CH₂O(CH₂)ₙ-, or -CH₂O(CH₂)ₙOC₂H₄-, n represents an integer of 1 to 5, and Q¹ represents F or -OCH₃.]

## Description

### Technical Field

The present invention relates to a diagnostic agent for cardiac function.

### Background Art

Positron emission tomography (PET) has been applied to various diagnoses. As a probe that can be used in the PET method, for example,

Patent Literature 1 discloses a compound suitable for detecting mitochondrial Complex-I.

### Citation List

### Patent Literature

[Patent Literature 1] PCT International Publication No. WO2014/30709

### Summary of Invention

### Technical Problem

The use of the compound disclosed in Patent Literature 1 for the purpose of diagnosing cardiac function has not been reported to date.

Conventionally, measurement by electrocardiogram and diagnosis by blood biochemical examination have been used as means for early detection of cardiac dysfunction. However, diagnosis by electrocardiogram requires that an electrocardiographic abnormality occur during the measurement period, making it an examination method with many missed diagnoses. In recent years, examinations by continuous monitoring using Holter electrocardiographs and the like have been spreading, but while this is a simple examination, the patient must wear the device for 24 hours or more, so the burden on the patient from long-duration examinations cannot be ignored. In blood biochemical examinations, the release into the blood of enzymes such as creatine kinase (CK) and aspartate aminotransferase (AST) accompanying myocardial cell destruction in myocardial infarction and the like occurs for approximately 24 hours from immediately after onset, making pinpoint examination necessary. In addition, these enzyme activities may show changes similar to those of myocardial infarction even in symptoms originating from skeletal muscle, and there have been challenges with these as indicators of myocardial dysfunction.

Diagnostic imaging related to the heart is dominated by ultrasound, CT, and MRI, but since all of these are evaluation methods for morphological evaluation and motion of the myocardium and physical quantities of the amount of blood discharged, they are suitable for measurement in a state where symptoms have progressed to a late stage of disease, but are not evaluation methods suitable for early detection.

Nuclear medicine examinations typified by PET include those using rubidium (⁸²Rb), ammonia ([¹³N]NH₃), and water ([¹⁵]H₂O) for cardiac examination, but these primarily measure changes in myocardial blood flow distribution due to myocardial ischemia and the like, and do not measure the biochemical function of the myocardium that directly leads to pathological diagnosis and assessment of therapeutic effects. Examination of myocardial function by PET also includes measurement of myocardial oxygen consumption using [¹¹C]Acetate, but since the half-life of ¹¹C is as short as only 20 minutes, it can only be used at PET facilities equipped with a cyclotron and has not become widespread. As a PET probe targeting the heart, [¹⁸F]FDG is used for confirming the viability of myocardial cells after myocardial infarction and for examination of cardiac sarcoidosis by utilizing the characteristic of [¹⁸F]FDG that it tends to distribute in inflammation, but since the tissue accumulation of [¹⁸F]FDG is influenced by blood glucose concentration, an overnight fasting period before the examination is required, and diabetic patients with high blood glucose concentrations are difficult to include as examination subjects.

In view of the circumstances described above, an object of the present invention is to provide a diagnostic agent for cardiac function that can sensitively diagnose functional changes in the heart.

### Solution to Problem

The present invention relates to a diagnostic agent for cardiac function containing a compound represented by General Formula (1-0) (hereinafter also referred to as a "compound (1-0)") as an active component.

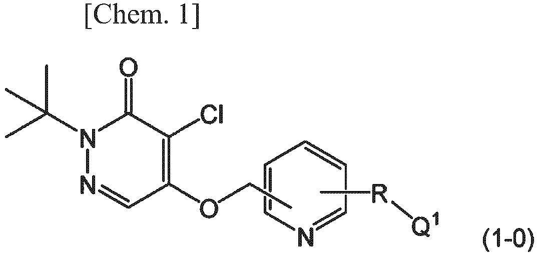

In General Formula (1-0), R represents -O(CH₂)ₙ-, - O(CH₂)ₙOC₂H₄-, -CH₂O(CH₂)ₙ-, or -CH₂O(CH₂)ₙOC₂H₄-, n represents an integer of 1 to 5, and Q¹ represents F or -OCH₃.

It is known that the compound (1-0) can be used for detecting mitochondrial complex I (hereinafter also referred to as "MC-I"). The diagnostic agent for cardiac function according to the present invention accumulates in the heart and the amount of the agent accumulated is proportional to the MC-I activity of the heart, and therefore the agent is suitably used for diagnosing cardiac function. In addition, as shown in the examples to be described below, the diagnostic agent for cardiac function according to the present invention can diagnose cardiac function based on the detection of MC-I activity even in a state where no changes in biochemical indicators, morphological indicators, and the like are observed. Accordingly, the diagnostic agent according to the present invention can sensitively diagnose functional changes in the heart. In addition, along with this, it is also possible to diagnose functional changes in the heart at an early stage.

In the above-described diagnostic agent, Q¹ may be ¹⁸F or - O¹¹CH₃. Accordingly, the above-described compound becomes capable of releasing positrons. The positrons released from the above-described compound immediately bind to electrons to release γ-rays (annihilation radiation). By measuring these γ-rays with a device used for positron emission tomography (PET method), the above-described compound accumulated in the heart can be imaged quantitatively and over time. That is, the compound can also be used as a labeled compound for the PET method.

The present invention can also be regarded as a method for diagnosing cardiac function, including: a step of administering the above-described diagnostic agent to a subject; a step of detecting the compound (1-0) accumulated in the heart; and a step of quantitatively analyzing an amount of compound (1-0) accumulated in the heart.

The present invention can further be regarded as a compound represented by General Formula (1-0) for use in diagnosis of cardiac function. The present invention can also be regarded as use of a compound represented by General Formula (1-0) in the manufacture of a diagnostic agent for cardiac function.

The diagnostic agent according to the present invention can sensitively diagnose functional changes in the heart, and therefore can also be used for evaluating side effects of a drug on the heart. That is, the diagnostic agent according to the present invention can also be regarded as an agent for evaluating side effects of a drug on the heart.

The present invention encompasses, for example, the following inventions.
[1] A diagnostic agent for cardiac function, containing a compound represented by General Formula (1-0) as an active component. 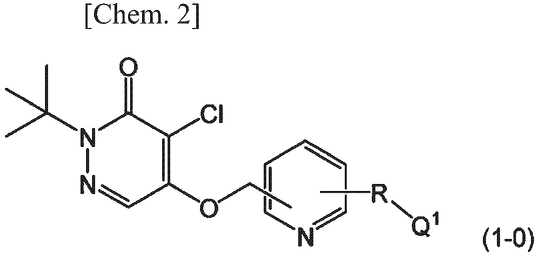 [In General Formula (1-0), R represents -O(CH₂)ₙ-, - O(CH₂)ₙOC₂H₄-, -CH₂O(CH₂)ₙ-, or -CH₂O(CH₂)ₙOC₂H₄-, n represents an integer of 1 to 5, and Q¹ represents F or -OCH₃.]
[2] An agent for evaluating side effects of a drug on the heart, containing a compound represented by General Formula (1-0) as an active component. 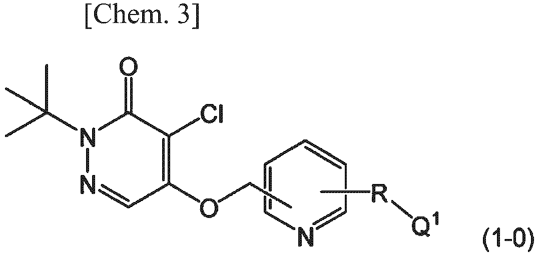 [In General Formula (1-0), R represents -O(CH₂)ₙ-, - O(CH₂)ₙOC₂H₄-, -CH₂O(CH₂)ₙ-, or -CH₂O(CH₂)ₙOC₂H₄-, n represents an integer of 1 to 5, and Q¹ represents F or -OCH₃.]
[3] The agent according to [1] or [2], wherein the active component is a compound represented by General Formula (1-0'). 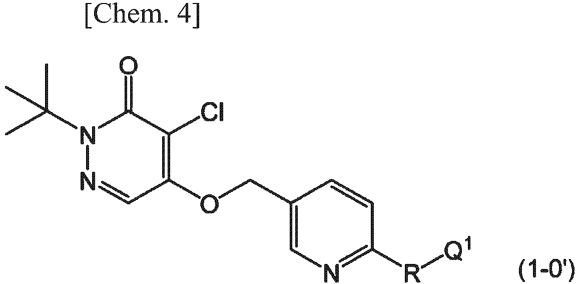 [In General Formula (1-0'), R, n, and Q¹ are synonymous with R, n, and Q¹ in General Formula (1-0).]
[4] The agent according to any one of [1] to [3], wherein the active component is a compound represented by General Formula (1-0"). 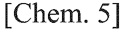 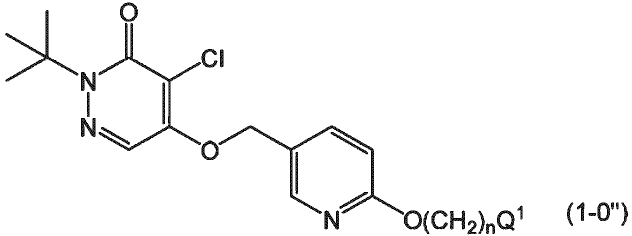 [In General Formula (1-0"), n and Q¹ are synonymous with n and Q¹ in General Formula (1-0).]
[5] The agent according to any one of [1] to [4], wherein the active component is a compound represented by Formula (1) below. 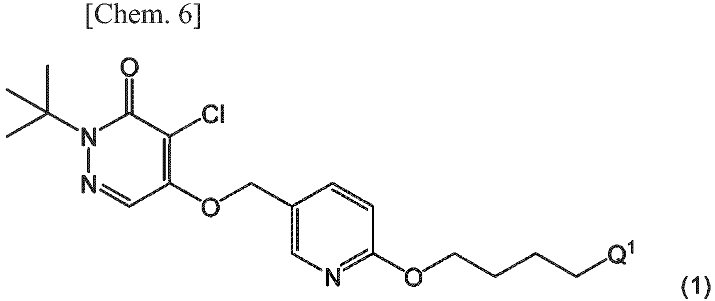 [In Formula (1), Q¹ is synonymous with Q¹ in General Formula (1-0).]
[6] The agent according to any one of [1] to [5], wherein Q¹ is ¹⁸F or -O¹¹CH₃.
[7] A method for diagnosing cardiac function of a subject, comprising:
   a step of administering the agent according to any one of [1] to [6] to a subject,
   a step of detecting the active component accumulated in the heart, and
   a step of quantitatively analyzing an amount of the active component accumulated in the heart.
[8] A method for evaluating side effects of a drug on the heart, comprising:
   a step of administering the drug to a subject,
   a step of administering the agent according to any one of [1] to [6] to the subject to which the drug has been administered,
   a step of detecting the active component accumulated in the heart, and
   a step of quantitatively analyzing an amount of the active component accumulated in the heart.
[9] The agent according to any one of [1] to [6] for use in diagnosis of cardiac function.
[10] The agent according to any one of [1] to [6] for use in evaluation of side effects of a drug on the heart.
[11] Use of the active component or agent according to any one of [1] to [6] in the manufacture of a diagnostic agent for cardiac function.
[12] Use of the active component or agent according to any one of [1] to [6] in the manufacture of an agent for evaluating side effects of a drug on the heart.

### Advantageous Effects of Invention

According to the present invention, it is possible to provide a diagnostic agent for cardiac function that can sensitively diagnose functional changes in the heart.

### Brief Description of Drawings

FIG. 1 (A) is a graph showing the amount of [¹⁸F]BCPP-BF accumulated in the heart (amount of radioactivity accumulated (SUV)). FIG.1 (B) is a graph showing the amount of [¹⁸F]FDG accumulated in the heart (amount of radioactivity accumulated (SUV)).
FIG. 2 (A) is a graph showing the amount of [¹⁸F]BCPP-BF accumulated in the heart (amount of radioactivity accumulated (SUV)). FIG. 2 (B) is a graph showing the amount of [¹⁸F]BMS accumulated in the heart (amount of radioactivity accumulated (SUV)).
FIG. 3 is a graph showing the amount of [¹⁸F]BCPP-BF accumulated in the heart (amount of radioactivity accumulated (SUV)).

### Description of Embodiments

Hereinafter, embodiments for carrying out the present invention will be described in detail. However, the present invention is not limited to the following embodiments.

The diagnostic agent for cardiac function according to the present embodiment (hereinafter also simply referred to as a "diagnostic agent") contains a compound represented by General Formula (1-0) as an active component. Note that, in the present specification, unless otherwise specified, each atom includes all isotopes.

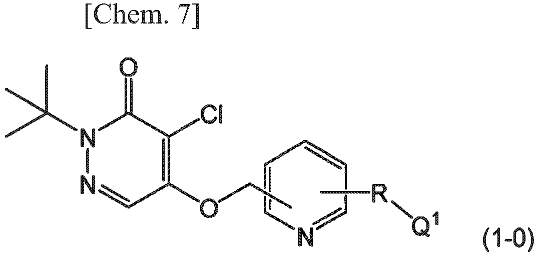

In the compound (1-0), R represents -O(CH₂)ₙ-, - O(CH₂)ₙOC₂H₄-, -CH₂O(CH₂)ₙ-, or -CH₂O(CH₂)ₙOC₂H₄-. R is preferably -O(CH₂)ₙ- or -O(CH₂)ₙOC₂H₄-, and more preferably - O(CH₂)ₙ-.

In the compound (1-0), n is an integer of 1 to 5, preferably an integer of 2 to 5, more preferably an integer of 3 to 5, and still more preferably 4.

In the compound (1-0), Q¹ is F or -OCH₃, and preferably ¹⁸F or - O¹¹CH₃. Since the compound (1-0) in which Q¹ is ¹⁸F or -O¹¹CH₃ can release positrons, it is suitable as a labeled compound (PET probe) for use in a PET method. In addition, in a case where Q¹ is -O¹¹CH₃, the half-life is as short as 20 minutes, so it is also possible to perform measurement multiple times on the same subject in one day. In a case where Q¹ is ¹⁸F, the half-life is 110 minutes, which is longer than that of -O¹¹CH₃, so it is possible to lengthen the time for one measurement.

The binding site of -OCH₂- bonded to the pyridazine ring and the binding site of R in the pyridine ring are not particularly limited, but it is preferable that the binding site of -OCH₂- bonded to the pyridazine ring is at position 5 of the pyridine ring and the binding site of R is at position 2 of the pyridine ring. A compound represented by General Formula (1-0') shown below (hereinafter also referred to as a "compound (1-0')") has a structural formula in which the binding site of -OCH₂-bonded to the pyridazine ring is at position 5 of the pyridine ring and the binding site of R is at position 2 of the pyridine ring.

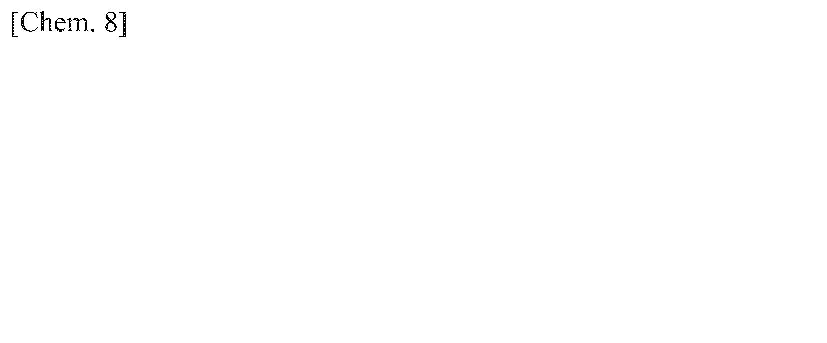

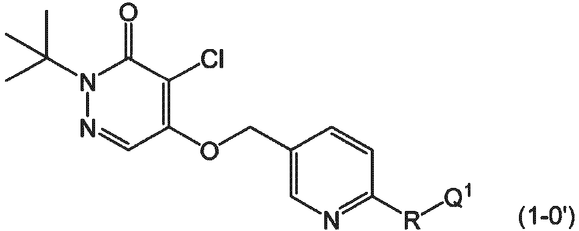

In General Formula (1-0'), R, n, and Q¹ are synonymous with R, n, and Q¹ in General Formula (1-0).

In view of making it more suitable for diagnosing cardiac function, the compound (1-0) is preferably a compound represented by General Formula (1-0") (hereinafter also referred to as a "compound (1-0")"), and more preferably a compound represented by Formula (1) (hereinafter also referred to as a "compound (1)").

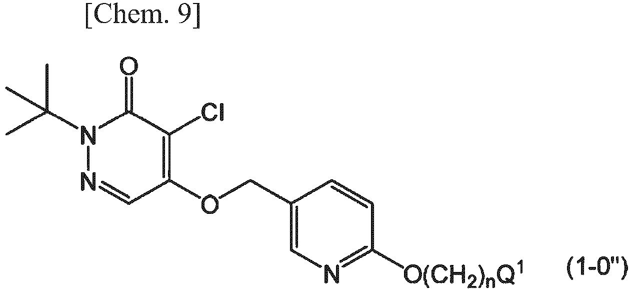

In General Formula (1-0''), n and Q¹ are synonymous with n and Q¹ in General Formula (1-0).

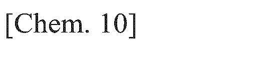

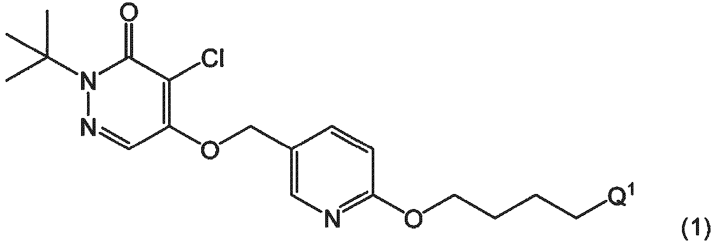

In Formula (1), Q¹ is synonymous with Q¹ in General Formula (1-0).

The compound (1-0) can be synthesized, for example, from a corresponding precursor. The same applies to the compound (1-0'), the compound (1-0''), and the compound (1).

Examples of precursors corresponding to the compound (1-0) include a compound represented by General Formula (2-0) below (hereinafter also referred to as a "compound (2-0)"). Examples of precursors corresponding to the compound (1-0'), the compound (1-0''), and the compound (1) include compounds in which, in the compound (2-0), R, the binding site of -OCH₂- bonded to the pyridazine ring in the pyridine ring, and the binding site of R are the same as those in the compound (1-0'), the compound (1-0"), and the compound (1).

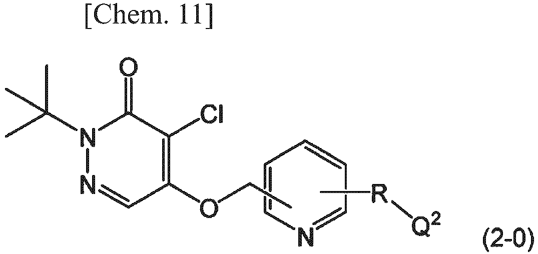

In General Formula (2-0), R is synonymous with R in General Formula (1-0). Q² represents a leaving group (a substituted sulfonyloxy group, a halogen atom, or a hydroxyl group, etc.).

Examples of substituted sulfonyloxy groups include a tosyloxy group (-OTs), a methanesulfonyloxy group (-OMs), a trifluoromethanesulfonyloxy group (-OTf), and a nitrobenzenesulfonyloxy group (-ONs), and -OTs is preferably used.

Examples of halogen atoms include fluorine, chlorine, bromine, and iodine.

A precursor can be synthesized through, for example, a method disclosed in PCT International Publication No. WO2014/30709.

Since the compound (1-0) accumulates in the heart specifically for MC-I, the accumulation amount changes in correlation with the degree of cardiac function. That is, the accumulation amount of compound (1-0) decreases when the cardiac function deteriorates, and the accumulation amount of compound (1-0) increases when the cardiac function is enhanced. Accordingly, the diagnostic agent according to the present embodiment is suitably used for diagnosing cardiac function through measurement of the accumulation amount of compound (1-0). The deterioration of cardiac function may be, for example, associated with a disease, disorder, dysfunction, or the like of the heart (for example, the myocardium or cardiovascular system). Note that the diagnosis of cardiac function can also be referred to as evaluation of cardiac function.

The diagnostic agent according to the present embodiment can be used, through the diagnosis of cardiac function, for example, in a method for screening subjects with deteriorated cardiac function (for example, group health examinations), a method for evaluating side effects of a drug on the heart, and a method for observing cardiac function over time (for example, follow-up observation of symptoms causing cardiac dysfunction, confirmation of therapeutic effects, or prognosis prediction). Accordingly, the diagnostic agent for cardiac function according to the present embodiment can also be regarded as, for example, an agent for evaluating side effects of a drug on the heart and an agent for evaluating the therapeutic effect on symptoms causing cardiac dysfunction.

The measurement of the accumulation amount of compound (1-0) is not limited to this, and can be carried out such that, for example, the compound (1-0) is bound to a fluorescent dye or the like, or labeled with a single photon nuclide (such as ¹²³I, ^{99m}Tc) or a positron nuclide to form a labeled compound, and the label is detected. The positron labeling can be performed, for example, by setting Q¹ of the compound (1-0) to -O¹¹CH₃ or ¹⁸F. In the case of the positron labeling, biodistribution of the compound (1-0) can be quantitatively imaged over time by measuring annihilation radiation with a device used for a PET method.

The diagnostic agent according to the present embodiment can be produced, for example, by dissolving the compound (1-0) in an arbitrary buffer solution. In this case, the diagnostic agent according to the present embodiment may be provided as a solution and contain other components such as a surfactant, a preservative, and a stabilizer in addition to the buffer component.

The method for diagnosing cardiac function according to the present embodiment includes: a step of administering the diagnostic agent according to the present invention to a subject; a step of detecting the compound (1-0) accumulated in the heart; and a step of quantitatively analyzing an amount of compound (1-0) accumulated in the heart.

The method for evaluating side effects of a drug on the heart according to the present embodiment includes a step of administering a drug to a subject, and can be carried out in the same manner as the above-described diagnostic method except that the subject in the above-described diagnostic method is a subject to which the drug has been administered. The drug may be any drug.

Examples of subjects include humans, monkeys, mice, and rats, but are not limited thereto.

The method for administering a diagnostic agent to a subject is not particularly limited as long as the compound (1-0) reaches the heart, but is usually intravenous administration.

The dose of a diagnostic agent is not particularly limited as long as it is a sufficient dose to detect the compound (1-0) in the heart, but may be appropriately set according to subjects to which it is administered and methods for detecting the compound (1-0). For example, in a case where the compound (1-0) is detected with a device used for a PET method using a diagnostic agent containing a compound (1-0) in which Q¹ is ¹⁸F or -O¹¹CH₃, the dose (hereinafter also referred to as an "amount of radioactivity administered") of the diagnostic agent may be 1 MBq/kg body weight to 1,000 MBq/kg body weight. The specific radioactivity of the compound (1-0) may be 10 to 10,000 GBq/µmol. In addition, the amount of radioactivity administered of a diagnostic agent depends on the sensitivity of a PET camera used and the volume of an individual subject, but about 200 to 500 MBq/kg body weight thereof is administered as 0.1 to 0.5 mL of a physiological saline solution for rodents (mice and rats). In a case of non-human primates (monkeys), 40 to 200 MBq/kg body weight thereof is administered as 0.5 to 2 mL of physiological saline, and in a case of humans, 2 to 10 MBq/kg body weight thereof is administered as 1 to 5 mL of a physiological saline solution.

The method for detecting the compound (1-0) accumulated in the heart is not particularly limited, and can be carried out according to well-known methods. For example, in a case where a diagnostic agent containing a compound (1-0) in which Q¹ is ¹⁸F or -O¹¹CH₃ is used, the compound (1-0) can be detected through a PET method. The measurement method in the PET method is not particularly limited, and can be carried out according to well-known methods. In addition, for example, as a measurement method in the PET method, dynamic measurement may be performed for 60 minutes immediately after administering a diagnostic agent, or PET measurement may be performed for 10 to 20 minutes after administering a diagnostic agent and waiting for 30 to 40 minutes to allow sufficient accumulation of the compound (1-0) in the heart.

The method for quantitatively analyzing the amount of compound (1-0) accumulated in the heart is not particularly limited, and can be carried out according to well-known methods. For example, the following method is exemplified. First, an accumulated image of the compound (1-0) obtained through a PET method and a morphological image of the heart obtained through CT measurement or the like are superimposed to identify a PET image of the heart. Next, a region of interest on the PET image of the heart is set, and a value normalized by the amount of radioactivity administered and the body weight of an individual subject is defined as an amount of compound (1-0) accumulated in the heart. In addition, an image obtained by a PET method in which a probe capable of detecting the heart is used may be used instead of the morphological image of the heart.

The diagnostic method according to the present embodiment may further include a step of comparing the quantitatively analyzed accumulation amount of compound (1-0) with a reference value to diagnose cardiac function.

The reference value may be appropriately set depending on the purpose of diagnosis. For example, in a case where the diagnostic method according to the present embodiment is carried out in a group health examination, the reference value may be a normal range previously determined from distribution of the accumulation amount of compound (1-0) in a plurality of the same kinds of subjects. In this case, whether or not the cardiac function of a specific subject is normal can be diagnosed depending on whether or not the quantitatively analyzed value of the accumulation amount of the specific subject is within the normal range.

In addition, for example, in a case where the diagnostic method according to the present embodiment is performed on subjects suffering from symptoms that cause cardiac dysfunction (for example, diabetes, lipid metabolism disorder, myocardial infarction, cardiac sarcoidosis, etc.) for follow-up observation of the symptoms, confirmation of therapeutic effects, prognosis prediction, or the like, the reference value may be a measurement result of the accumulation amount of compound (1-0) of the subject at a certain point in time (for example, at a point in time of being healthy, during initial diagnosis, at the beginning of treatment, at the end of treatment, etc.).

Furthermore, for example, in a case where the diagnostic method according to the present embodiment is carried out in the evaluation of side effects of a drug on the heart, the reference value may be a measurement result of the accumulation amount of compound (1-0) before the subject to which the drug is to be administered takes the drug. In this case, when the quantitatively analyzed value of the accumulation amount in the subject who has taken the drug is smaller than the reference value, it can be determined that the drug has a side effect on the heart.

### Examples

Hereinafter, the present invention will be described in more detail based on examples. However, the present invention is not limited thereto.

### [Test Example 1: Synthesis of PET Probe]

[¹⁸F]BCPP-BF represented by the following formula was synthesized through the method disclosed in Non Patent Literature (J. Labelled Comp. Radiopharm., 2013, Vol. 56, No. 11, pp. 553-561). The final product obtained had a radiochemical purity of 99% or more and a specific radioactivity of 43.8 to 103.9 GBq/µmol.

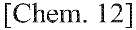

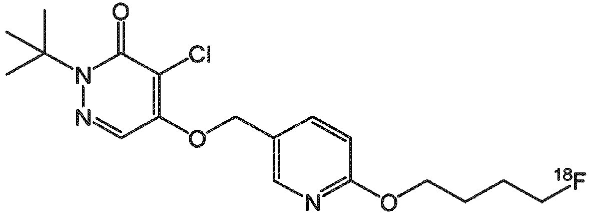

In addition, as controls, [¹⁸F]BMS-747158-02 (2-tert-butyl-4-chloro-5-[4-(2-fluoroethoxymethyl)-benzyloxy]-2H-pyridazin-3-one: hereinafter referred to as "[¹⁸F]BMS"), which is known as a PET probe that recognizes mitochondrial Complex-I, and ¹⁸F-fluorodeoxyglucose ([¹⁸F]FDG), which is known as a PET probe targeting the heart, were prepared. The radiochemical purity of [¹⁸F]BMS was 99% or more and the specific radioactivity was 36.3 to 76.1 GBq/µmol. The radiochemical purity of [¹⁸F]FDG was 99% or more.

### [Test Example 2: Evaluation of Cardiac Function in Type 2 Diabetes Model Rats]

### (Type 2 Diabetes Model Rats)

Male Zucker Lepr^{fa}/Lepr^{fa} rats (hereinafter also referred to as "diabetic rats") that develop pathological conditions similar to type 2 diabetes of human adults were purchased from Charles River Laboratories Japan, Inc. and used for PET measurement at 5 weeks and 26 weeks of age. As controls, male Zucker Lepr^{fa}/+ rats (hereinafter also referred to as "normal rats") were purchased from Charles River Laboratories Japan, Inc. and used.

### (PET Measurement)

The rats were anesthetized with isoflurane and fixed in a gantry of an animal PET camera (SHR-38000, manufactured by Hamamatsu Photonics K.K.). After 15-minute transmission measurement was carried out for absorption correction, about 20 MBq/0.5 mL of [¹⁸F]BCPP-BF was administered to the rats via the tail vein and 60-minute emission measurement was carried out. A region of interest was set on the heart, and the amount of [¹⁸F]BCPP-BF accumulated in the region of interest was calculated. Subsequently, the calculated accumulation amount was normalized by the amount of radioactivity administered and the body weight of each individual and defined as an amount of [¹⁸F]BCPP-BF accumulated in the heart (amount of radioactivity accumulated (SUV)). As a control, the amount of [¹⁸F]FDG accumulated in the heart (amount of radioactivity accumulated (SUV)) was measured in the same manner except that [¹⁸F]FDG was used instead of [¹⁸F]BCPP-BF.

### (Measurement of Blood Glucose Concentration)

Blood was collected from each rat immediately after the PET measurement, and the blood glucose concentration was measured. The blood glucose concentration was measured using a biochemical automatic analyzer (7180 manufactured by Hitachi High-Tech Corporation).

### (Results)

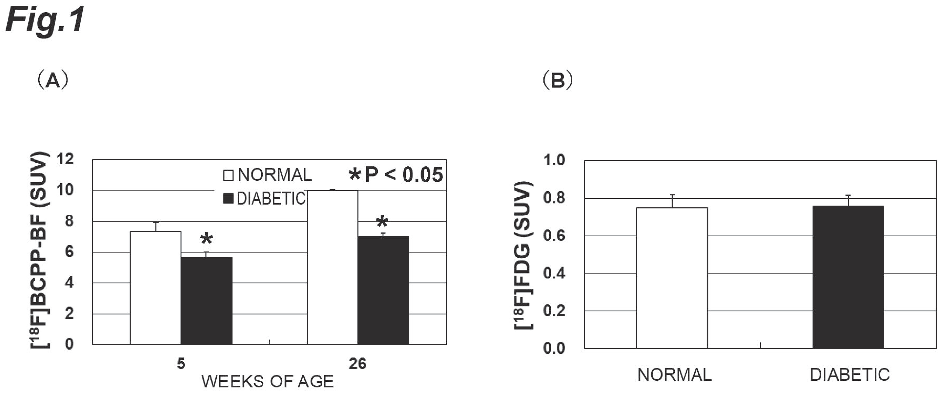

FIG. 1(A) is a graph showing the amount of [¹⁸F]BCPP-BF accumulated in the heart (amount of radioactivity accumulated (SUV)). FIG. 1(B) is a graph showing the amount of [¹⁸F]FDG accumulated in the heart (amount of radioactivity accumulated (SUV)). FIG. 1(B) shows the results measured in 26-week-old rats. In FIG. 1, "*" indicates that the difference between the data of normal rats and the data of diabetic rats of the same week of age was statistically significant (p < 0.05).

It has been reported that when diabetes develops and a state of high blood glucose continues, blood vessels are damaged and complications such as heart disease, kidney disease, blindness, and foot amputation are more likely to occur, and that when diabetic patients suffer attacks such as myocardial infarction or angina pectoris, the risk of heart failure and subsequent death increases (Circulation R., 2020, Vol. 126, pp. 1501-1525).

As shown in FIG. 1(A), PET measurement using [¹⁸F]BCPP-BF was able to detect abnormalities in cardiac function in diabetic rats as a decrease in mitochondrial function at 5 weeks and 26 weeks of age. The blood glucose concentration at 26 weeks of age was 135.3±17.9 mg/dL in normal rats and 588.3±29.9 mg/dL in diabetic rats, and the diabetic rats had reached a diabetic state showing a statistically significantly high value. On the other hand, the blood glucose concentration at 5 weeks of age was 146.0 ± 13.8 mg/dL in normal rats and 171.4±24.2 mg/dL in diabetic rats, showing a slightly elevated tendency, but the difference was not statistically significant. This indicates that PET measurement using [¹⁸F]BCPP-BF can detect abnormalities in cardiac function due to diabetes from an extremely early stage.

On the other hand, as shown in FIG. 1(B), PET measurement using [¹⁸F]FDG could not detect a significant difference relative to normal rats even in 26-week-old diabetic rats that had reached a diabetic state showing a statistically significantly high value.

According to a prior study using the same model rats as in this test example, no difference was observed in morphological changes and fibrosis of the heart evaluated from hearts excised from 14-week-old normal rats and diabetic rats (Am. J. Physiol. Heart Circ. Physiol., 2007, Vol. 293, H292-H298), and no difference between normal rats and diabetic rats was observed in oxygen metabolism and the rate of ATP production from glucose evaluated in hearts excised at 12 weeks of age (Am. J. Physiol. Heart Circ. Physiol., 2005, Vol. 288, H2102-H2110). Furthermore, no difference between normal rats and diabetic rats was observed in the heart rate measured in vivo at 14 weeks of age or in the metabolic rate of ATP transfer via creatine kinase (CK), which is a biochemical indicator of myocardial energy metabolism (CK flux) (Physiol. Rep., 2015, 3(1), e12248).

From these results, it can be seen that by evaluating mitochondrial function through PET measurement using [¹⁸F]BCPP-BF, the deterioration of cardiac function due to diabetes can be detected with extremely high sensitivity and at an extremely early stage that cannot be detected by biochemical indicators, morphological indicators, and the like.

### [Test Example 3: Evaluation of Side Effects on the Heart Associated with Administration of a Drug (Acetaminophen)]

Acetaminophen (hereinafter also referred to as "APAP"), a representative antipyretic analgesic widely used throughout the world, was administered to normal rats, and the effects (side effects) on cardiac function were evaluated.

### (PET Measurement)

100 mg/kg or 300 mg/kg of APAP was administered intravenously via the tail vein of the rats. As a control, rats to which only the solvent was similarly administered intravenously were prepared. The rats were subjected to PET measurement 24 hours after administration of APAP or the solvent. The procedure for PET measurement is the same as in Test Example 2. In addition, as a control, measurement was similarly performed using [¹⁸F]BMS instead of [¹⁸F]BCPP-BF.

### (Results)

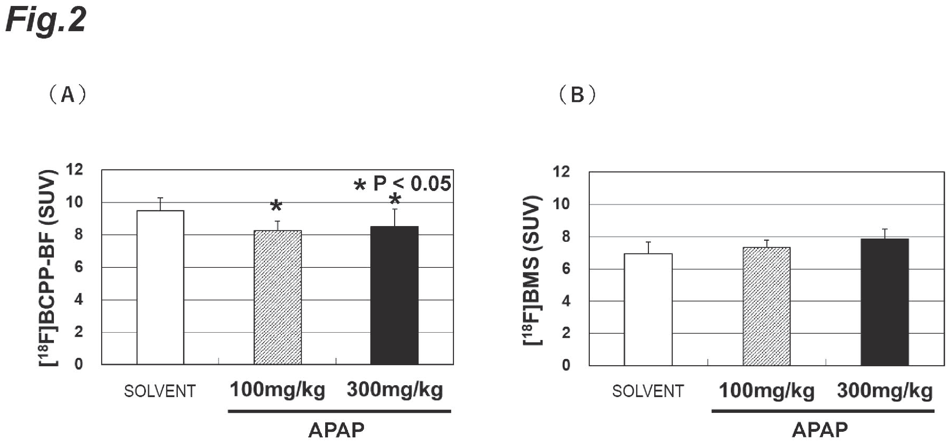

FIG. 2(A) is a graph showing the amount of [¹⁸F]BCPP-BF accumulated in the heart (amount of radioactivity accumulated (SUV)). FIG. 2(B) is a graph showing the amount of [¹⁸F]BMS accumulated in the heart (amount of radioactivity accumulated (SUV)). In FIG. 2, "*" indicates that the difference from the data of the control rats (rats to which the solvent was administered) was statistically significant (p < 0.05).

It has been reported that overdose of APAP causes acute liver dysfunction (Semin. Liver Dis., 2008, Vol. 28, pp. 142-152) and acute kidney dysfunction (J. Am. Soc. Nephrol., 1995, Vol. 6, pp. 48-53). In prior studies by the present inventors, it has been shown that these disorders can be detected at an early stage by PET measurement targeting rats using [¹⁸F]BCPP-BF (EJNMMI Res., 2016, Vol. 6, pp. 82, Japanese Patent No. 7005126).

On the other hand, it is thought that the effects on cardiac function had been overlooked until now, perhaps because the damage that overdose of APAP causes to liver function and kidney function is so severe. As shown in FIG. 2(A), PET measurement using [¹⁸F]BCPP-BF revealed that 24 hours after intravenous administration of 100 mg/kg and 300 mg/kg of APAP, a significant effect was also exerted on the mitochondrial function of the heart.

On the other hand, as shown in FIG. 2(B), PET measurement using [¹⁸F]BMS could not detect a significant difference in the mitochondrial function of the heart 24 hours after APAP administration under the same conditions.

According to a prior study, a transient significant increase in blood pressure was observed 2 minutes after intravenous administration of 125 mg/kg of APAP, but no significant difference from the solvent administration group was observed after 3 minutes. On the other hand, no effect was observed on heart rate (J. Cardiovasc. Pharmacol. Therapeut., 2003, Vol. 8, pp. 277-284). An increase in the expression of inflammation-related genes has been confirmed in the hearts of rats excised 24 hours after oral administration of a high dose of 1,000 mg/kg of APAP, but the histological necrotic changes observed in the liver were not observed in the myocardium (Yonsei Med. J., 2012, Vol. 53, pp. 172-180).

From these results, it can be seen that by evaluating mitochondrial function through PET measurement using [¹⁸F]BCPP-BF, the effects (side effects) of a drug (APAP) on cardiac function can be detected with extremely high sensitivity.

### [Test Example 4: Evaluation of Side Effects on the Heart Associated with Administration of a Drug (Doxorubicin)]

Doxorubicin (hereinafter also referred to as "DOX") is an anticancer drug that inhibits DNA synthesis to suppress the proliferation of cancer cells and has the effect of shrinking tumors (Med. Res. Rev., 2014, Vol. 34, pp. 106-135). Although DOX is used for the treatment of various cancers such as malignant lymphoma, lung cancer, gastrointestinal cancer, breast cancer, bladder tumor, and osteosarcoma, it is known to have side effects of myocardial damage and heart failure such as shortness of breath, difficulty breathing, chest pain, leg edema, and tachycardia, and therefore it is contraindicated in patients with cardiac dysfunction or a history thereof, and biomarkers for early detection of the cardiotoxicity of DOX are sought. In this test example, DOX was administered to normal rats, and the effects (side effects) on cardiac function were evaluated.

### (PET Measurement)

5 mg/kg or 20 mg/kg of DOX was administered intravenously via the tail vein of the rats. As a control, rats to which only the solvent was similarly administered intravenously were prepared. The rats were subjected to PET measurement 0.5 hours, 24 hours, and 96 hours after administration of DOX or the solvent. The procedure for PET measurement is the same as in Test Example 2.

### (Results)

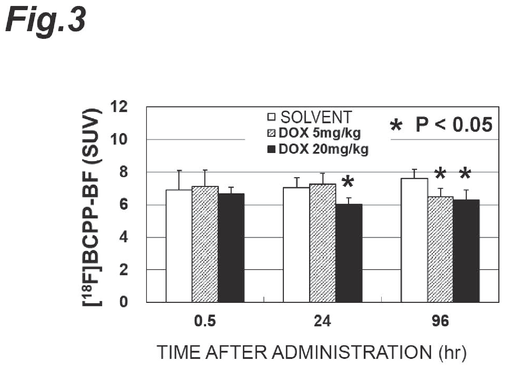

FIG. 3 is a graph showing the amount of [¹⁸F]BCPP-BF accumulated in the heart (amount of radioactivity accumulated (SUV)). In FIG. 3, "*" indicates that the difference from the data of the control rats (rats to which the solvent was administered) at the same time after administration was statistically significant (p < 0.05).

As shown in FIG. 3, PET measurement using [¹⁸F]BCPP-BF detected a significant decrease in mitochondrial function starting 24 hours after DOX administration in high-dose (20 mg/kg) rats. In addition, a significant decrease in mitochondrial function was also detected 96 hours after DOX administration in low-dose (5 mg/kg) rats.

According to a prior study, 24 hours after intravenous administration of 20 mg/kg of DOX, abnormalities in cardiac function could not be detected by CK and troponin I (TnI), which are blood markers of myocardial abnormality (12). In addition, even 24 hours after intravenous administration of DOX at a much higher dose (40 mg/kg) than our dose, abnormalities in cardiac function could not be detected by CK, TnI, lactate dehydrogenase (LDH: Lactate Dehydrogenase), and fatty acid-binding protein 3 (FABP3: Fatty acid-binding protein 3) (PLoS ONE, 2012, Vol. 7, e38867). Furthermore, even at 3 weeks after repeated intravenous administration of 3 mg/kg of DOX every week, changes in cardiac function such as heart rate and cardiac output, TnI, LDH, and furthermore changes in myocardial metabolism measured using hyperpolarized [1-¹³C]pyruvate and [2-¹³C]pyruvate could not be detected (Commun. Biol., 2020, Vol. 3, pp. 692).

From these results, it can be seen that by evaluating mitochondrial function through PET measurement using [¹⁸F]BCPP-BF, the effects (side effects) of a drug (DOX) on cardiac function can be detected with extremely high sensitivity.

## Claims

1. A diagnostic agent for cardiac function, comprising a compound represented by General Formula (1-0) as an active component. 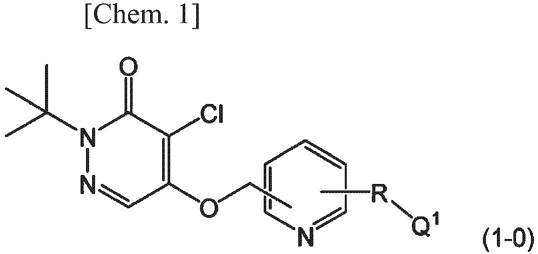 [In General Formula (1-0), R represents -O(CH₂)ₙ-, - O(CH₂)ₙOC₂H₄-, -CH₂O(CH₂)ₙ-, or -CH₂O(CH₂)ₙOC₂H₄-, n represents an integer of 1 to 5, and Q¹ represents F or -OCH₃.]

2. An agent for evaluating side effects of a drug on the heart, comprising a compound represented by General Formula (1-0) as an active component. 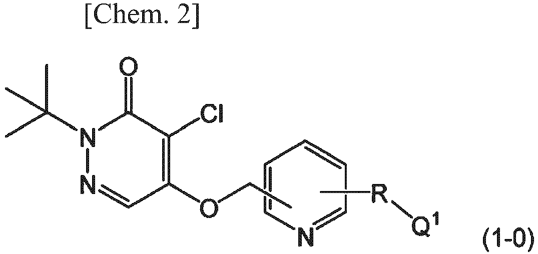 [In General Formula (1-0), R represents -O(CH₂)ₙ-, - O(CH₂)ₙOC₂H₄-, -CH₂O(CH₂)ₙ-, or -CH₂O(CH₂)ₙOC₂H₄-, n represents an integer of 1 to 5, and Q¹ represents F or -OCH₃.]

3. The agent according to claim 1 or 2, wherein the active component is a compound represented by General Formula (1-0'). 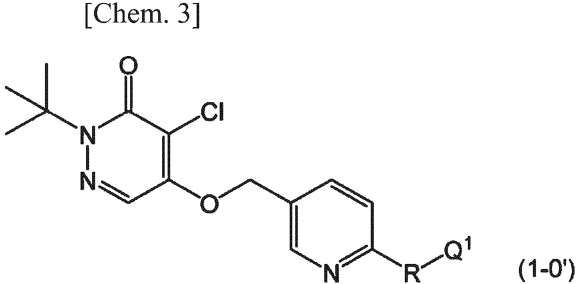 [In General Formula (1-0'), R, n, and Q¹ are synonymous with R, n, and Q¹ in General Formula (1-0).]

4. The agent according to claim 1 or 2, wherein the active component is a compound represented by General Formula (1-0"). 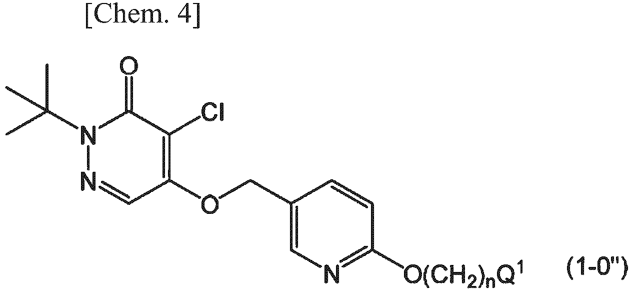 [In General Formula (1-0"), n and Q¹ are synonymous with n and Q¹ in General Formula (1-0).]

5. The agent according to claim 1 or 2, wherein the active component is a compound represented by Formula (1) below. 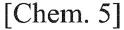 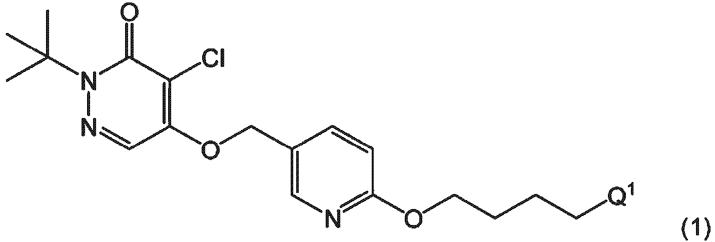 [In Formula (1), Q¹ is synonymous with Q¹ in General Formula (1-0).]

6. The agent according to claim 1 or 2, wherein Q¹ is ¹⁸F or - O¹¹CH₃.
